# EUROPEAN PATENT SPECIFICATION

(11) **EP 2 728 011 B1**
(45) Date of publication and mention of the grant of the patent: **25.10.2017**
(21) Application number: 11842461.3
(22) Date of filing: 27.07.2011
(51) Int. Cl.: C12P 21/06, A23J 3/34

(54) **OPTIMISED METHOD FOR OBTAINING ANGIOTENSIN CONVERTING ENZYME INHIBITORY PEPTIDES, INHIBITORY PEPTIDES AND FOOD CONTAINING SAME**
OPTIMIERTES VERFAHREN ZUR GEWINNUNG VON PEPTIDEN ZUR HEMMUNG ANGIOTENSIN-UMWANDELNDER ENZYME, HEMMERPEPTIDE UND NAHRUNGSMITTEL DAMIT
PROCÉDÉ OPTIMISÉ POUR L'OBTENTION DE PEPTIDES INHIBITEURS DE L'ENZYME DE CONVERSION DE L'ANGIOTENSINE, PEPTIDES INHIBITEURS ET ALIMENT LES COMPRENANT

(30) Priority: 15.06.2011 ES 201131001
(43) Date of publication of application: 07.05.2014
(73) Proprietor: Queizuar, S.L., 15822 Touro (A Coruña) (ES)
(72) Inventor: PEREIRA RAMOS, Benigno, E-15822 Touro (A Coruña) (ES); PEREIRA RODRIGUEZ, Angel, E-15822 Touro (A Coruña) (ES); ESTEVEZ TELLE, Natalia, E-15822 Touro (A Coruña) (ES); FUCIÑOS GONZALEZ, Juan Pablo, E-15822 Touro (A Coruña) (ES); RUA RODRIGUEZ, Maria Luisa, E-15822 Touro (A Coruña) (ES); PASTRANA CASTRO, Lorenzo, E-15822 Touro (A Coruña) (ES)
(74) Representative: Carvajal y Urquijo, Isabel
(86) International application number: PCT/ES2011/070551
(87) International publication number: WO 2012/172129

(56) References cited:
- EP-A1- 1 967 524
- EP-A1- 1 967 524
- WO-A1-2004/098310
- WO-A1-2006/025731
- WO-A2-2007/004876
- WO-A2-2007/004876
- DE-A1-102008 032 828
- US-A1- 2004 191 857
- HERNANDEZ-LEDESMA B ET AL: "Effect of beta-lactoglobulin hydrolysis with thermolysin under denaturing temperatures on the release of bioactive peptides", JOURNAL OF CHROMATOGRAPHY, ELSEVIER SCIENCE PUBLISHERS B.V, NL, vol. 1116, no. 1-2, 26 May 2006 (2006-05-26), pages 31-37, XP024967370, ISSN: 0021-9673, DOI: 10.1016/J.CHROMA.2006.03.006 [retrieved on 2006-05-26]
- HERNANDEZ-LEDESMA B ET AL.: 'Effect of beta-lactoglobulin hydrolysis with thermolysin under denaturing temperatures on the release of bioactive peptides.' JOURNAL OF CHROMATOGRAPHY vol. 1116, no. 1-2, 26 May 2006, NL., pages 31 - 37, XP024967370
- WANG LEI ET AL.: 'Effect of enzyme type and hydrolysis conditions on the in vitro angiotensin 1-converting enzyme inhibitory activity and ash content of hydrolysed whey protein isolate. International' JOURNAL OF FOOD SCIENCE & TECHNOLOGY vol. 45, no. 4, April 2010, pages 807 - 812, XP055136159
- PHELAN M ET AL.: 'Casein-derived bioactive peptides: Biological effects, industrial uses, safety aspects and regulatory status.' INTERNATIONAL DAIRY JOURNAL vol. 19, no. 11, 01 November 2009, GB, pages 643 - 654, XP026562819
- ABUBAKAR A ET AL.: 'Structural Analysis of New Antihypertensive Peptides Derived from Cheese Whey Protein by Proteinase K Digestion.' JOURNAL OF DAIRY SCIENCE vol. 81, no. 12, 01 December 1998, US., pages 3131 - 3138, XP027111216

## Description

### Field of the Invention

The present invention relates to an optimized method for obtaining angiotensin converting enzyme (ACE) activity inhibitory peptides from whey, ACE inhibitory peptides and a functional food comprising said peptides.

The preceding objects are included within the food sector, especially in the field of the milk industry.

### Background of the Invention

The literature describes many methods for releasing bioactive peptides from milk proteins. Some of them are based on fermenting those and other materials, the others are based on enzymatic hydrolysis both with animal proteases (pepsin, trypsin and chemotrypsin) and plant proteases (papain and bromelain) or microbial proteases (proteinase K or thermolysin). The advantages of the last methods relate to the milder reaction conditions in which they take place (Otte *et al.,* 1997) and to the different specificity for the residues involved in the peptide bond facilitating the isolation and characterization of the generated peptides. In contrast, the main drawback of enzymatic hydrolysis is that it often leads to the production of a strong bitter taste due to the presence of hydrophobic amino acid residues in the peptide obtained.

EP 1967524 A1 discloses a two-step procedure to obtain hydrolysates with ACE-inhibitory peptides using a whey protein concentrate rich in β-lactoglobulin as starting material. The content of α-lactoalbumin in said concentrate is below 0.01% according to the characterization showed on page 8 by means of RP-HPLC. Herein, the hydrolysis is conducted under a control of pH.

Hernandez-Ledesma B. et al ("Effect of β-lactoglobulin hydrolysis with thermolysin under denaturing temperatures on the release of bioactive peptides", Journal of Chromatography, Elsevier Science Publishers B.V, NL, vol. 1116, no. 1-2, pages 31-37, 2006.05.26) disclose the hydrolysis of bovine β-lactoglobulin A (β-Lg A) with thermolysin under non-denaturing and heat-denaturing conditions, wherein a total of 25 peptides were identified by HPLC-MS/MS and wherein the hydrolysis was conducted at 37ºC for 5-30 minutes, and using 10 mM of CaCl2 buffer (pH 8.0).

Among milk proteins, caseins are very susceptible to hydrolysis due to their open flexible structure. For this reason, a large number and variety of antihypertensive peptides of this origin are described (Yamamoto *et al.*, 1999). In contrast, the generation of antihypertensive activity from whey proteins depends to a large extent on the protease used and on the hydrolysis conditions.

The most widely used digestive proteases are trypsin, pepsin and chemotrypsin. Trypsin has been successfully used in the production of ACE inhibitory peptides from whey proteins. This may be due to the specificity of ACE for substrates or competitive inhibitors with Lys or Arg at the C-terminal end (López-Fandiño *et al.*, 2006). Bovine β-Lg contains many bonds which are theoretically susceptible to pepsin. However, it is resistant to the action of said enzyme (Reddy *et al.*, 1988) due to the compact protein structure which conceals the hydrophobic amino acids susceptible to hydrolysis (Brownlow *et al.*, 1997). Partial heat denaturation of β-Lg involves the appearance of bonds susceptible to pepsin. In contrast, α-La is sensitive to the action of pepsin (Schmidt and van Marwijk, 1993), but times longer than 3 hours are required for the release of ACE inhibitory peptides (Pihlanto-Leppälä *et al.*, 2000). Lastly, chemotrypsin quickly hydrolyzes α-La and β-lug is hydrolyzed more slowly (Schmidt and Poll, 1991). The combination of the three digestive enzymes, pepsin, trypsin and chemotrypsin, has allowed releasing small peptides with ACE inhibitory activity (Pihlanto-Leppälä *et al.,* 2000).

The main advantage of using digestive enzymes for obtaining antihypertensive peptides is that it simulates the physiological conditions of the organism in the release of peptides with biological activity. The main drawback is the lower availability and the high price of these enzymes compared with microbial enzymes.

The most widely used microbial enzymes for the hydrolysis of whey proteins are proteinase K and thermolysin. The hydrolysates obtained after the action of these enzymes on the whey protein concentrates have ACE inhibition percentages greater than 95% (Saito *et al.,* 1997). Hydrolysates with thermolysin and proteinase K, for example, had a strong ACE inhibitory activity (Hernández-Ledesma *et al.*, 2002; 2006; Abubakar *et al.*, 1998). Experiments with mixtures of microbial enzymes have in any case been performed for this purpose.

Table 1 shows some of the ACE inhibitory peptides identified in the whey hydrolysates hydrolyzed with different enzymes and the corresponding IC₅₀ values.

**Table 1: ACE inhibitory peptides obtained by the enzymatic hydrolysis of whey proteins.**

| Peptide | Sequence | Enzyme | IC₅₀ (µM) | Reference |
|---|---|---|---|---|
| β-Lg f(78-80) | IPA | Proteinase k | 141 | Abubakar *et al.,* (1998) |
| β-Lg f(81-83) | VFK | Trypsin | 1029 | Pihlanto-Leppälä *et al.* (2000) |
| β-Lg f(22-25) | LAMA | Trypsin | 556 | Pihlanto-Leppälä *et al.* (2000) |
| *β*-Lg (f 142-148) | ALPMHIR | Trypsin | 42.6 | Mullally *et al.* (1997) |
| β-Lg f(32-40) | LDAQSAPLR | Trypsin | 635 | Pihlanto-Leppälä *et al.* (2000) |
| *β*-Lg f(103-105) | LLF | Thermolysin | 79.8 | Hernández-Ledesma *et al.* (2002) |
| *β*-Lg f(58-61) | LKQW | Thermolysin | 34.7 | Hernández-Ledesma *et al.* (2002) |
| β-Lg f(94-100) | VLDTDYK | Pepsin, trypsin, chemotrypsin | 946 | Pihlanto-Leppälä *et al.* (2000) |
| β-Lg f(106-111) | CMENSA | Pepsin, trypsin, chemotrypsin | 788 | Pihlanto-Leppälä *et al.* (2000) |
| β-Lg f(15-19) | VAGT | Pepsin, trypsin, chemotrypsin | 1054 | Pihlanto-Leppälä *et al.* (2000) |
| β-Lg f(142-146) | ALPMH | Pepsin, trypsin, chemotrypsin | 521 | Pihlanto-Leppälä *et al.* (2000) |
| β-Lg (f 102-105) | YLLF | Pepsin, trypsin, chemotrypsin | 172 | Mullally *et al.* (1997) |
| β-Lg (f 46-53) | LKPTPEGN | Thermolysin | >2700 | Hernandez-Ledesma *et al.* (2002) |
| β-Lg (f 122-125) | LVRT | Thermolysin | 2470 | Hernández-Ledesma *et al.* (2002) |
| *α-La* f(50-53) | YGLF | Pepsin | 733 | Mullally *et al.* (1997) |
| *α-La* f(50-52) | YGL | Pepsin, trypsin, chemotrypsin | 409 | Pihlanto-Leppälä *et al.* (2000) |
| *α*-*La* f(104-108) | WLAHK | Trypsin | 77 | Pihlanto-Leppälä *et al.* (2000) |
| *α-La* f(99-108) | VGINYWLAH K | Trypsin | 327 | Pihlanto-Leppälä *et al.* (2000) |
| BSA f(221-222) | FP | Proteinase k | 315 | Abubakar *et al.* (1998) |
| BSA f(208-216) | ALKAWSVAR | Trypsin | 3 | Chiba and Yoshikawa (1991) |

Despite extensive research conducted for obtaining antihypertensive peptides from whey proteins, as of today the processes designed have barely been optimized both in terms of enzyme consumption and of the search for mixtures of proteases which allow reducing the necessary enzyme concentration in the mixture for obtaining peptides with suitable IC₅₀ values.

The proposed invention consists specifically of designing a method which, by means of the suitable selection and proportion of two proteases having complementary action and specificity, allows reducing the enzyme concentration necessary for obtaining a hydrolysate providing a specific IC₅₀ value and inhibition percentage.

### Description of the Invention

The present application relates to an optimized method for obtaining angiotensin converting enzyme (hereinafter, ACE) activity inhibitory peptides. The method optimally combines two proteases to perform substrate hydrolysis and achieves a reduction in the amount of enzymes used for obtaining the peptides. Therefore, greater ACE inhibition results are achieved with a lower concentration of enzymes.

According to a first aspect, an optimized method is provided using whey as the raw material in which it includes an ultrafiltration of said whey in order to obtain a whey concentrate, an enzymatic hydrolysis of this whey concentrate for a minimum time of 30 minutes, protein hydrolysate ultrafiltration and ACE inhibitory peptide separation and production, which comprises:
a. selecting and adding a mixture having a suitable proportion and amount of two microbial proteases and having complementary action to said whey concentrate for the hydrolysis thereof, in which said proportion of enzyme/substrate ranges between 0.5 and 3 enzyme units/mg of whey protein concentrate in order to obtain peptides with an ACE inhibition percentage greater than 70% and an IC₅₀ index of 30 to 100 µg/ml. The optimized method is based on applying a statistical mathematical algorithm of factorial experimental design from that described by Akhnazarova and Kafarov (1982) which allows quantitatively describing the effect that the process variables, such as the enzyme concentration or enzyme/substrate ratio in the reaction mixture and the composition of a mixture of enzymes, have on the production of antihypertensive peptides. Since the algorithm also allows identifying the interactions existing between the studied variables, it is possible to more precisely define the operating conditions which maximize the production of peptides or minimize enzyme consumption.

In an operating mode, this method is applied, obtaining, from the preceding algorithm, a polynomial model of the type Y = b₀ + b_{1X1} + b_{2X2} + b_{12X2} + b_{11X12} + b_{22X22}, wherein Xₙ and Xₙₘ represent the variables studied and their interactions, respectively; bₙ and bₙₘ are the weight of each variable and their interaction in the end result, respectively, and Y is the target variable (production of peptides, IC₅₀ value, enzyme use).

According to the method, the whey is subjected to an ultrafiltration obtaining a whey concentrate with a protein content between 55 and 65 g/l, preferably about 60 g/l, which involves a concentration factor between 2.5 to 3 times. Said whey concentrate is then subjected to enzymatic hydrolysis.

It has been taken into account in the hydrolysis that the comparison of the different protein hydrolysates obtained will be based on the use of the IC₅₀ (50% inhibitory concentration) index which is defined as the peptide concentration (µg/ml) necessary for inhibiting 50% of the ACE activity in the assay.

In an optional embodiment, the whey concentrate is hydrolyzed using proteases having analytical grade purity, i.e., having a degree of purity such as that required in analytical laboratories, selected from the following group: thermolysins, proteinases K, trypsins and chemotrypsins.

According to another option, the whey concentrate is hydrolyzed using proteases having "technical grade purity", i.e., the degree of purity commonly used in the industry, since they have the advantage that they allow greatly lowering the costs of the method in the scaling phase if compared with the enzymes with high purity. These proteases having technical grade purity are selected from the group consisting of alcalases and neutrases. By means of this option, inhibition percentages of 80%, greater than those obtained with the enzymes having high purity can be achieved in some cases (assays with alcalase and neutrase).

According to a preferred embodiment, the whey concentrate is hydrolyzed by means of the complementary and synergistic action of the proteases having high purity and proteases having technical grade purity, for example, by means of one of the following protease combinations: thermolysin/proteinase k, alcalase/thermolysin and thermolysin/neutrase. For such combinations, the assayed proportions range between 15 and 85% of each of the enzymes in the mixture. In an embodiment of the invention, an IC₅₀ of 37 µg/ml was obtained by using a thermolysin/proteinase K protease combination, each at 50%. In another embodiment, an IC₅₀ of 57 µg/ml was obtained by using the 85% neutrase / 15% thermolysin combination.

High inhibition percentages greater than 70% are thus obtained depending on the assayed conditions. It is thus possible (within this range) to modify both the total amount of added enzymes and the ratio thereof at will to achieve a desired value of ACE inhibition power.

To that end, the total protease enzyme activity units which must be added to the system can be reduced up to 7 times and at the same time those with a lower cost can be selected, or work can be performed in the most convenient conditions (temperature conditions, for example). All this constitutes a revaluation task since the raw material (whey) has little economic value, and precisely the cost of external resources and that of technological processing itself are what determine the profit margin.

In relation to the protein hydrolysates obtained after enzymatic hydrolysis, the following facts have been taken into account: on one hand, it is generally known that hydrolysates containing peptides with a molecular weight less than 1 kDa have IC₅₀ values less than those containing only peptides with a high molecular weight or than those containing mixtures of peptides with a high or low molecular weight and, on the other hand, it is necessary to remove the proteases, the non-hydrolyzed proteins and also the large peptides produced.

Therefore, it is necessary to purify the hydrolysates. According to an optional embodiment, this purification is performed by means of an ultrafiltration membrane. The use of ultrafiltration has been shown to greatly reduce the IC₅₀ value of the mixtures of hydrolysates from many sources.

Ultrafiltration is carried out in two steps: first, a first ultrafiltration in which the hydrolysates are fractioned using membranes with a 10 kDa cut-off and the permeate obtained was then fractioned through a second ultrafiltration using membranes with a 1 kDa cut-off. A permeate with extracts of 1 kDa peptides is thus obtained.

After ultrafiltration, peptides with greater ACE inhibitory activity are selected. To that end a separation is performed by means of high performance liquid chromatography (HPLC) with reverse phase columns. Therefore, on one hand, inhibitory peptides having higher purity are obtained, and on the other, the separated peptides can be sequenced and identified (by means of MALDI-TOF, for example).

According to a second aspect, ACE inhibitory peptides obtained by applying the method described above are supplied. Said inhibitory peptides have an IC₅₀ index of between 30 and 100 µg/ml and ACE inhibition percentages greater than 70%. These peptides are preferably used in the production of functional foods.

According to a third aspect of the invention, a functional food comprising ACE activity inhibitory peptides obtained from whey by means of the enzymatic method described above is supplied.

### Embodiment of the Invention

The following embodiment only seeks to illustrate how the present invention is carried out and it must in no way be taken into account for defining the scope of the invention.

A volume of whey from a cheese factory, ultrafiltered until achieving a protein concentration of 60 g/l, is subjected to enzymatic hydrolysis with different thermolysin/proteinase k combinations and in different enzyme/substrate ratios according to the design of the following table.

| E: Enzyme concentration (units/ml) | R: Thermolysin/proteinase k ratio |
|---|---|
| 172 | 0.85 |
| 172 | 0.15 |
| 51 | 0.85 |
| 51 | 0.15 |
| 197.06 | 0.5 |
| 25.94 | 0.5 |
| 111.5 | 0.5 |

The reaction was performed at 60°C and pH = 8 in 100 mL Pyrex bottles loaded with a final reaction volume of 32 mL which were closed with screw-on caps to prevent possible losses on evaporation and were under continuous stirring to favor the mixing. The two proteases were dissolved in a 0.02 M Tris-HCl-0.01 M CaCl₂ buffer to a concentration of 1 mg/mL prior to the hydrolysis. The hydrolysis was prolonged for at least 6 hours.

Applying a mathematical algorithm consisting of a rotatable factorial design of the order of 2 to the hydrolysis results in the mentioned conditions expressed as IC₅₀ values allowed obtaining the following statistically significant model: %IC₅₀ = 24.9 +1.86E-3.38R² which allows knowing, for any combination of the variables R (protease ratio in the reaction mixture) and E (enzyme activity units in the reaction mixture), the %IC₅₀ which will be obtained in the hydrolysis or, complementarily, for a required IC₅₀ value, the value of enzyme units added to the reaction (variable E) and the ratio of the two proteases (variable R) that could be used.

Even though the thermolysin/proteinase k combination is used in this example, other combinations such as alcalase/thermolysin K, thermolysin/neutrase can also be used.

In the conditions of interest defined according to the model, the hydrolysates obtained are ultrafiltered. In a first pass, the hydrolysates are filtered through a membrane with a 10 kDa cut-off to obtain a first hydrolysate permeate with a molecular weight less than 10 kDa and, in a second pass, this first permeate is filtered through a membrane with a 1 kDa cut-off to obtain a second permeate with a molecular weight less than 1 kDa and thus remove the high molecular weight fractions.

Said second permeate is then subjected to a step of separation by means of high performance liquid chromatography (HPLC) with reverse phase columns to select the peptide extracts with greater inhibitory activity.

The peptides thus obtained can be used in the production of functional foods, for example, in the production of dairy products and in the production of drinks and beverages.

## Claims

1. A method for obtaining ACE activity inhibitory peptides wherein it includes the steps of whey ultrafiltration for obtaining a whey concentrate, enzymatic hydrolysis, hydrolysate ultrafiltration and inhibitory peptide separation, **characterized in that** it comprises:
selecting and adding a mixture having two microbial proteases and having complementary action to said whey concentrate for the hydrolysis thereof, wherein said proportion of enzyme/substrate ranges between 0.5 and 3 enzyme units/mg of whey protein in order to obtain peptides with an ACE inhibition percentage greater than 70% and an IC₅₀ index of 30 to 100 µg/ml.

2. The method according to claim 1, **characterized in that** the mixture of proteases is selected from one of the following combinations: thermolysin-proteinase k, thermolysin-neutrase and alcalase-thermolysin, whereby the proportion of each of the proteases in the mixture ranges between 15 and 85%.

3. ACE inhibitory peptides obtained by means of the method defined according to claims 1 and 2.

4. ACE inhibitory peptides according to claim 3, **characterized in that** they have an IC₅₀ index between 30 and 100 µg/ml and ACE inhibition percentages greater than 70%.

5. ACE inhibitory peptides according to claims 3 and 4, **characterized in that** they are used in the production of functional foods.

6. A functional food comprising ACE inhibitory peptides according to claim 3.

## Patentansprüche

1. Verfahren zur Gewinnung von Peptiden zur Hemmung von ACE-Aktivität, wobei es die Schritte der Molkenultrafiltration zur Gewinnung eines Molkenkonzentrats, enzymatischen Hydrolyse, Ultrafiltration von Hydrolysat und des Abtrennens hemmender Peptide enthält, **dadurch gekennzeichnet, dass** es Folgendes umfasst:
Auswählen und Zugeben eines Gemisches mit zwei mikrobiellen Proteasen und komplementärer Wirkung auf das Molkenkonzentrat zur Hydrolyse davon, wobei das Verhältnis von Enzym/Substrat im Bereich zwischen 0,5 und 3 Enzymeinheiten/mg von Molkenprotein zur Gewinnung von Peptiden mit einem prozentualen Anteil der ACE-Hemmung von mehr als 70 % und einem IC₅₀-Index von 30 bis 100 µg/ml liegt.

2. Verfahren nach Anspruch 1, **dadurch gekennzeichnet, dass** das Gemisch von Proteasen aus einer der folgenden Kombinationen ausgewählt ist: Thermolysin-Proteinase K, Thermolysin-Neutrase und Alcalase-Thermolysin, wobei das Verhältnis jeder der Proteasen in dem Gemisch im Bereich zwischen 15 und 85 % liegt.

3. Peptide zur Hemmung von ACE, die mittels des Verfahrens nach Anspruch 1 und 2 gewonnen wurden.

4. Peptide zur Hemmung von ACE nach Anspruch 3, **dadurch gekennzeichnet, dass** sie einen IC₅₀-Index zwischen 30 und 100 µg/ml und einen prozentualen Anteil der ACE-Hemmung von mehr als 70 % aufweisen.

5. Peptide zur Hemmung von ACE nach Anspruch 3 und 4, **dadurch gekennzeichnet, dass** sie bei der Herstellung von funktionellen Lebensmitteln verwendet werden.

6. Funktionelles Lebensmittel nach Anspruch 3, das Peptide zur Hemmung von ACE umfasst.

## Revendications

1. Procédé pour l'obtention de peptides inhibiteurs de l'activité ECA dans lequel il comporte les étapes d'ultrafiltration de lactosérum pour obtenir un concentré de lactosérum, l'hydrolyse enzymatique, l'ultrafiltration d'hydrolysate et la séparation de peptides inhibiteurs, **caractérisé en ce qu'**il comprend :
la sélection et l'ajout d'un mélange ayant deux protéases microbiennes et ayant une action complémentaire audit concentré de lactosérum pour l'hydrolyse de celui-ci, dans lequel ladite proportion d'enzyme/substrat est comprise entre 0,5 et 3 unités/mg d'enzymes de protéine de lactosérum afin d'obtenir des peptides avec un pourcentage d'inhibition d'ECA supérieur à 70 % et un indice IC₅₀ de 30 à 100 µg/ml.

2. Procédé selon la revendication 1, **caractérisé en ce que** le mélange de protéases est choisi parmi l'une des combinaisons suivantes : la thermolysine-protéinase k, la thermolysine-neutrase et l'alcalase-thermolysine, dans lequel la proportion de chacune des protéases dans le mélange est comprise entre 15 et 85 %.

3. Peptides inhibiteurs d'ECA obtenus au moyen du procédé défini selon les revendications 1 et 2.

4. Peptides inhibiteurs d'ECA selon la revendication 3, **caractérisés en ce qu'**ils ont un indice IC₅₀ entre 30 et 100 µg/ml et des pourcentages d'inhibition d'ECA supérieurs à 70 %.

5. Peptides inhibiteurs d'ECA selon les revendications 3 et 4, **caractérisés en ce qu'**ils sont utilisés dans la production d'aliments fonctionnels.

6. Aliment fonctionnel comprenant des peptides inhibiteurs d'ECA selon la revendication 3.
